# EUROPEAN PATENT APPLICATION

(11) **EP 2 147 662 A1**
(43) Date of publication of application: **27.01.2010**
(21) Application number: 08013265.7
(22) Date of filing: 23.07.2008
(51) Int. Cl.: A61F 2/84, A61M 25/10

(54) **Stent delivery system**

(71) Applicant: Abbott Laboratories Vascular Enterprises Limited, Dublin 2 (IE)
(72) Inventor: Stockert, Gunter, 72076 Tübingen (DE); Bregulla, Rainer, 72336 Balingen (DE)
(74) Representative: Schmitz, Hans-Werner

(57) **Abstract**

Stent delivery system (1) comprising a catheter (2) having an inner tube (3) and an outer tube (4) surrounding the inner tube (3); a balloon (5) having a proximal end (6) fixed to the outer tube (4) a distal end (7) fixed to the inner tube (3) and an interior (10) including a proximal portion (10A) and distal portion (10B), a stent (8) disposed on said balloon (5) and being expandable from a delivery position to a deployed position by a contrast medium (9) adapted to be supplied to the interior (10) of said balloon (5), and a contrast medium distribution means (11) disposed in the interior (10) of said balloon (5) adapted to equally distribute the contrast medium (9) to both the proximal portion (10A) and the distal portion (10B) of the interior (10) of the balloon (5).

## Description

The present invention relates to a stent delivery system.

One of the most significant parameters of a stent delivery system to secure reliable stent retention during the expansion is the nature of how the balloon is expanded. Most stent delivery systems particularly those with balloon diameters over 6mm suffer from an inadequate or unequal opening nature/procedure of both the proximal and the distal portion of the interior of the balloon and consequently a stent movement or dislocation is caused therefrom, which results in an increased risk during the delivery of the stent into a lumen of a body vessel.

It is, therefore, an object of the present invention to provide a stent delivery system with an improved balloon opening nature which equally expands the distal and proximal portions of the balloon.

The solution of this object is achieved by the features of claim 1.

The stent delivery system according to the present invention comprises a catheter having an inner tube and an outer tube surrounding the inner tube, a balloon having a proximal end fixed to the outer tube, a distal end fixed to the inner tube and an interior including a proximal portion and distal portion. Further, the stent delivery system comprises a stent disposed on said balloon and being expandable from a delivery position to a deployed position by an inflation medium adapted to be supplied to the interior of said balloon, and a contrast medium distribution means disposed in the interior of said balloon adapted to equally distribute the contrast medium to both the proximal portion and the distal portion of the interior of the balloon.

By expanding the balloon and in particular the proximal and distal end portions thereof equally a so called "dogbone-expansion" of the balloon is assured and a reliable and improved retention of the stent disposed thereon compared to the common stent delivery systems can be achieved.

The dependent claims contain advantageous embodiments of the present invention.

In a first embodiment of the invention the contrast medium distribution means is the outer tube extended to the middle of the interior of the balloon.

In a second embodiment the contrast medium distribution means is the outer tube extended further to the distal portion of the balloon.

In a further embodiment of the present invention the outer tube comprises proximal and distal distribution holes.

In a further embodiment of the present invention the medium distribution means is a profiled tube between the markers.

In a further embodiment of the present invention the medium distribution means is a spiral tube on the inner tube, wherein the spiral tube can be configured as to constitute the markers.

Further features and advantages of the present invention will become apparent from the following description of preferred embodiments with reference to the appended drawings, wherein
Fig. 1 shows an enlarged cross sectional view of a distal portion of a stent delivery system according to a first embodiment of the present invention,
Fig. 2 shows an enlarged cross sectional view of a distal portion of a stent delivery system according to a second embodiment of the present invention,
Fig. 3 shows an enlarged cross sectional view of a distal portion of a stent delivery system according to a further embodiment of the present invention,
Fig. 3a, 3b, 3c each show an enlarged cross sectional view of the deflated balloon portion of the stent delivery system including a different profiled outer tube according to the present invention,
Fig. 4 shows an enlarged cross sectional view of a distal portion of a stent delivery system according to a further embodiment of the present invention,
Fig. 4a shows an enlarged view of the deflated balloon portion of the stent delivery system, and
Fig. 4b shows an enlarged cross sectional view of a distal portion of a stent delivery system according to a further embodiment of the present invention.

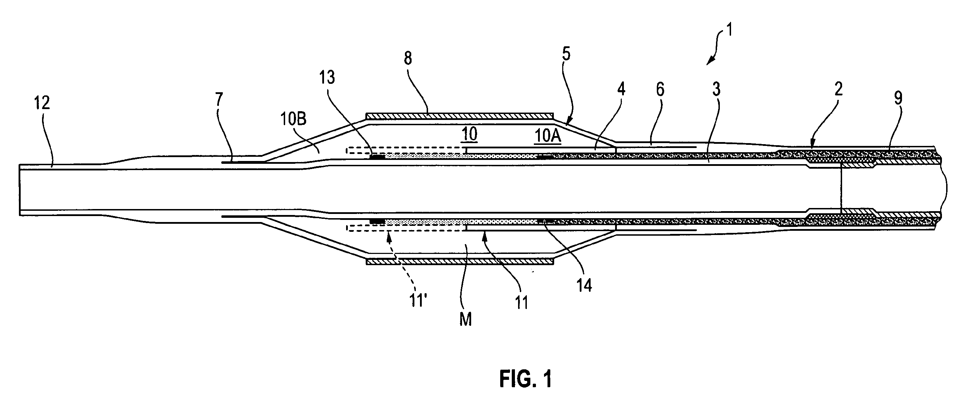

The enlarged cross sectional view of Fig. 1 illustrates a distal portion of a stent delivery system 1 according to a first embodiment of the present invention. As can be seen from Fig. 1 the stent delivery system 1 comprises a catheter 2 including an inner tube 3 and an outer tube 4 surrounding the inner tube 3. A proximal end 6 of a balloon 5 is fixed to said outer tube 4 whilst a distal end 7 of the balloon 5 is fixed to said inner tube 3 of the catheter 2. An interior 10 of the balloon 5 includes a proximal portion 10A and a distal portion 10B and a stent 8 being disposed on said balloon 5 shown in an inflated state. A contrast medium 9 can be supplied to the interior 10 of the balloon 5 to expand the stent 8 from a delivery position to a deployed position.

According to a first embodiment of the present invention the outer tube 4 serves as a contrast medium distribution means 11 which is extended to the middle M of the interior 10 of the balloon 5 and is adapted to equally distribute the contrast medium 9 to both the proximal portion 10A and the distal portion 10B of the balloon 5. Alternatively, as also illustrated in Fig. 1, the outer tube 4 serving as a contrast medium distribution means 11' can be extended further to the distal end portion 10B of the balloon 5. Due to these arrangements of the outer tube 4 an improved supply of the inflating contrast medium 9 can be equally occur in the proximal end 6 and distal end 7 of the balloon 5 and the above mentioned desired "dogbone-expansion" is achieved.

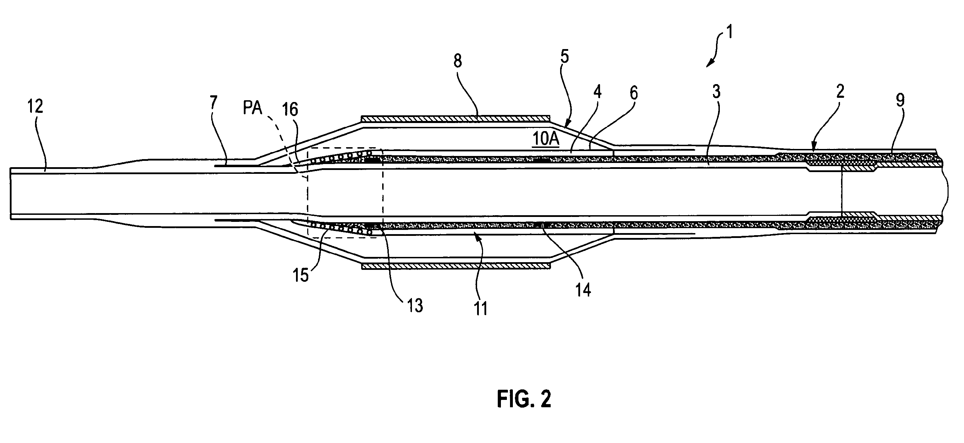

Fig. 2 shows an enlarged cross sectional view of a distal portion of the stent delivery system 1 according to a second embodiment of the present invention. The contrast medium distribution means 11, in this case the outer tube 4, is extended up to a distal cone 16 thereof and provided with a plurality of (proximal and preferably distal) distribution holes 15 for an improved flow of the contrast medium 9 into the distal end 7 of the balloon 5. In the same way another perforated area of the outer tube 4 can symmetrically be provided in the proximal end 6 of the balloon 5 for an improved flow of the contrast medium 9 in the proximal end 6 of the balloon 5. With the enhanced supply of contrast medium 9 via these perforated areas PA the desired "dogbone-expansion" of the balloon 5 can be achieved.

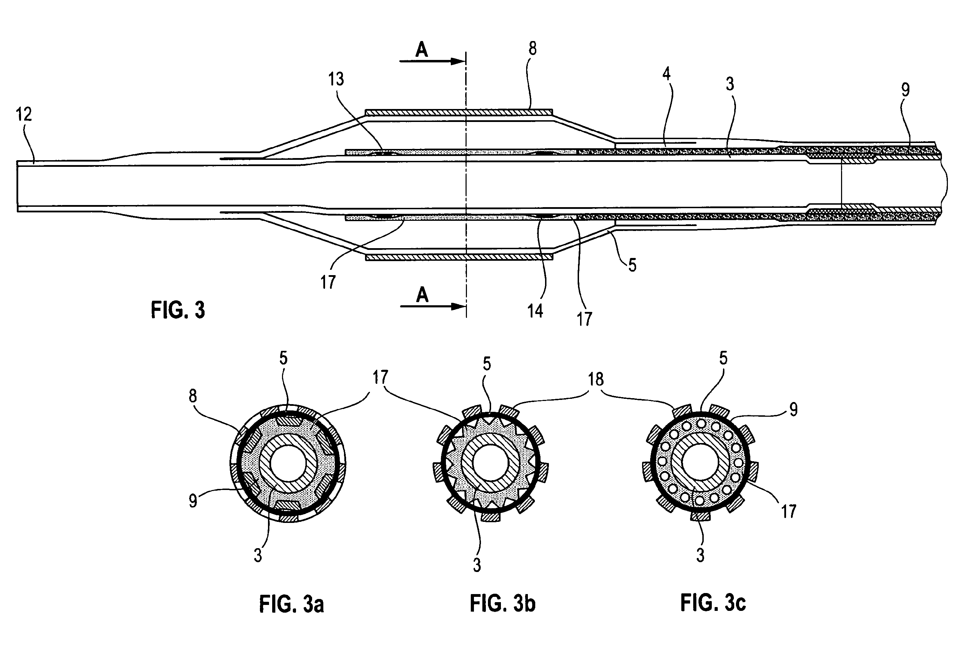

Fig. 3 shows an enlarged cross sectional view of a distal portion of the stent delivery system 1 in an inflated state according to a further embodiment of the present invention. In this embodiment the contrast medium distribution means 11 is a profiled tube 17 disposed on the inner tube 3 within the interior 10 of balloon 5. The contrast medium 9 supplied from the outer tube 4 flowing through the free cross sections of the profiled tube 17 will result in an equal radial expansion over the whole linear extension of the profiled tube 17 which extends beyond the markers 13, 14 into the proximal and distal regions of the balloon 5.

This profiled tube 17 can be formed in different profiles. Cross sectional views of examples of profiles when viewed into the direction of an arrow A in Fig. 3 are depicted in Fig. 3a, 3b and 3c in a deflated state of the balloon 5. As can be seen in Fig. 3a - 3c the profiled tube 17 is disposed on the inner tube 3 and the balloon 5 and the stent 8 are crimped thereon. Due to this arrangement of the profiled tube 17 between the distal and proximal ends 10B, 10A of the balloon 5 the desired "dogbone-expansion" of the balloon 5 is achieved.

As a matter of course it should be understood that the present invention is not limited to the profiles depicted in Fig. 3a - 3c but a variety of other forms of profiles can be employed as profiled tube 17 for the stent delivery system 1 of the present invention as well.

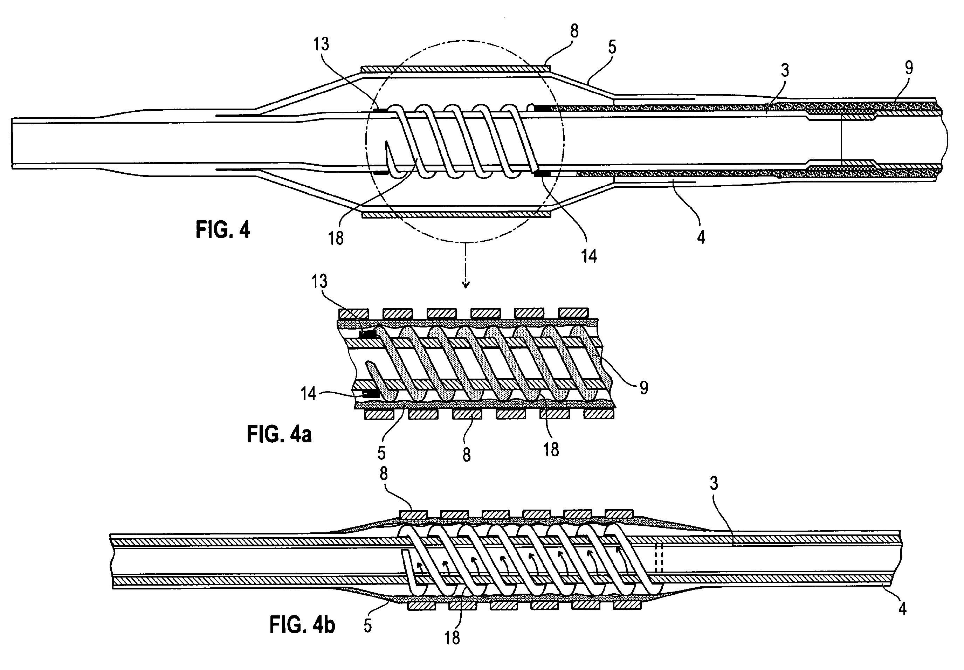

Fig. 4 illustrates an enlarged cross sectional view of a distal portion of a stent delivery system 1 according to a further embodiment of the present invention in an inflated state. In this embodiment a spiral tube 18, which can be a flat or round wire, is disposed within the balloon 5 between the markers 13, 14 to improve the supply of the contrast medium 9 flowing through this spiral tube 18 for the desired equal radial expansion of the balloon 5.

In Fig. 4a an enlarged partial view of a balloon portion of the stent delivery system 1 for a detailed illustration of the deflated state of the arrangement is depicted.

Fig. 4b shows an enlarged cross sectional view of a distal portion of a stent delivery system 1 according to a further embodiment of the present invention in a deflated state. In this embodiment the spiral tube 18 not only serves for the improved supply of the contrast medium 9 and desired expansion of the balloon 5 but also performs the function of the markers 13, 14, which as shown in the above embodiments are commonly disposed between the distal and proximal ends 10B, 10A of the balloon 5 to simplify the overall structure of the stent delivery system 1 of the present invention.

In addition to the written disclosure reference is herewith made explicitly to the disclosure of the invention in Fig. 1 to 4b.

### List of reference signs

- 1: stent delivery system
- 2: catheter
- 3: inner tube
- 4: outer tube
- 5: balloon
- 6: proximal end
- 7: distal end
- 8: stent
- 9: contrast medium
- 10: interior
- 10A: proximal portion
- 10B: distal portion
- 11, 11': contrast medium distribution means
- 12: tip
- 13, 14: marker
- 15: distribution hole
- 16: distal cone
- 17: profiled tube
- 18: spiral tube
- M: middle of the interior
- PA: perforated area

## Claims

1. Stent delivery system (1) comprising:
- a catheter (2) having:
· an inner tube (3) and
· an outer tube (4) surrounding the inner tube (3);
- a balloon (5) having:
· a proximal end (6) fixed to the outer tube (4),
· a distal end (7) fixed to the inner tube (3) and
· an interior (10) including a proximal portion (10A) and distal portion (10B);
- a stent (8) disposed on said balloon (5) and being expandable from a delivery position to a deployed position by a contrast medium (9) adapted to be supplied to the interior (10) of said balloon (5), and
- a inflation medium distribution means (11) disposed in the interior (10) of said balloon (5) adapted to equally distribute the contrast medium (9) to both the proximal portion (10A) and the distal portion (10B) of the interior (10) of the balloon (5).

2. Stent delivery system (1) of claim 1 wherein the contrast medium distribution means (11) is the outer tube (4) extended to the middle (M) of the interior (10) of said balloon 5.

3. Stent delivery system (1) of claim 1 wherein the contrast medium distribution means (11') is the outer tube (4) extended to the distal portion (10B) of said balloon 5.

4. Stent delivery system (1) of one of claims 1 to 3 wherein the outer tube (4) comprises proximal and distal distribution holes (15)

5. Stent delivery system (1) of claim 1 wherein the contrast medium distribution means (11) is a profiled tube (17) between markers (13, 14) on the inner tube (3).

6. Stent delivery system (1) of claim 1 wherein the contrast medium distribution means (11) is a spiral tube (18) on the inner tube (3)

7. Stent delivery system (1) of claim 6 wherein the spiral tube (18) is configured so to constitute as the markers (13, 14).
